# EUROPEAN PATENT SPECIFICATION

(11) **EP 4 734 823 B1**
(45) Date of publication and mention of the grant of the patent: **09.12.2026**
(21) Application number: 24735608.2
(22) Date of filing: 20.06.2024
(51) Int. Cl.: A61B 5/00, A61B 5/11, A46B 15/00, A61C 17/22

(54) **SYSTEM AND METHOD FOR DETECTING AND COMPENSATING TREMORS**
SYSTEM UND VERFAHREN ZUR ERKENNUNG UND KOMPENSATION VON TREMORES
SYSTÈME ET PROCÉDÉ DE DÉTECTION ET DE COMPENSATION DE TREMBLEMENTS

(30) Priority: 27.06.2023 EP 23181779
(43) Date of publication of application: 06.05.2026
(73) Proprietor: Koninklijke Philips N.V., 5656 AE Eindhoven (NL)
(72) Inventor: JOHNSON, Mark, Thomas, 5656 AG Eindhoven (NL); GERHARDT, Lutz, Christian, 5656 AG Eindhoven (NL); VAN DEN DUNGEN, Wilhelmus, Andreas, Marinus, 5656 AG Eindhoven (NL); MENA BENITO, Maria Estrella, 5656 AG Eindhoven (NL); RMAILE, Amir, Hussein, 5656 AG Eindhoven (NL)
(74) Representative: Philips Intellectual Property & Standards
(86) International application number: PCT/EP2024/067240
(87) International publication number: WO 2025/002972

(56) References cited:
- WO-A1-2016/133621
- WO-A1-2020/205557
- CN-A- 106 344 105
- ES-A1- 2 222 819
- US-A1- 2010 030 119
- US-A1- 2019 125 067
- US-A1- 2022 233 119
- US-B1- 6 259 224
- ANUPAM PATHAK ET AL: "A noninvasive handheld assistive device to accommodate essential tremor: A pilot study", MOVEMENT DISORDERS, vol. 29, no. 6, 27 May 2014 (2014-05-27), US, pages 838 - 842, XP055297140, ISSN: 0885-3185, DOI: 10.1002/mds.25796
- PATEL S ET AL: "Monitoring Motor Fluctuations in Patients With Parkinson's Disease Using Wearable Sensors", IEEE TRANSACTIONS ON INFORMATION TECHNOLOGY IN BIOMEDICINE, IEEE SERVICE CENTER, LOS ALAMITOS, CA, US, vol. 13, no. 6, 1 November 2009 (2009-11-01), pages 864 - 873, XP011345622, ISSN: 1089-7771, DOI: 10.1109/TITB.2009.2033471
- HUO WEIGUANG ET AL: "A Heterogeneous Sensing Suite for Multisymptom Quantification of Parkinson's Disease", vol. 28, no. 6, 1 June 2020 (2020-06-01), USA, pages 1397 - 1406, XP055904681, ISSN: 1534-4320, Retrieved from the Internet <URL:https://ieeexplore.ieee.org/ielx7/7333/9109394/09064818.pdf?tp=&arnumber=9064818&isnumber=9109394&ref=aHR0cHM6Ly9pZWVleHBsb3JlLmllZWUub3JnL2Fic3RyYWN0L2RvY3VtZW50LzkwNjQ4MTg=> DOI: 10.1109/TNSRE.2020.2978197

## Description

### FIELD OF THE INVENTION

This invention relates to the detection of tremors when using a hand-held device, and the compensation for such tremors.

### BACKGROUND OF THE INVENTION

Tremor is defined as a rhythmical, involuntary oscillatory movement of a body part. Tremors are the result of involuntary alternating contractions of reciprocally innervated muscles. Tremors are the most common movement disorder symptom, especially in the elderly population.

Tremors are likely to hamper many basic self-care activities, which require a good amount of motor coordination such as brushing, shaving, bathing etc. These activities are often referred as Activities of Daily Living (ADLs). This can lead to a major change in daily routine, absenteeism from work or even psychological issues.

Essential tremor (ET) is the most common cause of action tremor, with an estimated prevalence worldwide of 1 percent overall and approximately 5 percent in adults over the age of 60 years. The incidence of ET increases with age, although childhood and early adulthood presentations do occur, especially when ET is familial.

In context of oral health, poor motor coordination can lead to poor brushing quality, which can lead to suboptimal oral hygiene, which ultimately can lead to oral diseases. Therefore, it would be important in such cases to provide some means to minimize the physical disability due to tremors so that a person can perform the required physical activity with minimum discomfort.

The most common method for diagnosis of tremors is analysis of a patient's history and thorough physical examination along with targeted neurological assessment. The physical examination for tremor involves assessment of different characteristic of tremors such as affected body parts, position of body part, relation with body movements (at rest; during action) and frequency of tremors. Such characterization helps in grouping tremors according to their pathophysiology and aetiology, which in turn is highly relevant for choosing the most promising treatment option for tremors. The characteristic of different tremors and how they can be detected are discussed below.

### Orthostatic tremor

This is a postural tremor in the torso and lower limbs while standing, and it may also occur in the upper limbs. It is suppressed by walking. The tremor is high frequency (14 to 20 Hz) and synchronous among ipsilateral and contralateral muscles.

### Physiologic tremor

This is a predominantly bilateral, symmetrical action tremor. The tremor is again high frequency (10 to 12 Hz), with known causes (e.g., medications, hyperthyroidism, hypoglycemia).

### Essential tremor

This usually presents as a bilateral postural tremor of the hands in a frequency range 6 to 12Hz, followed by a kinetic and resting component.

### Parkinsonism Tremor

This is predominantly at rest and is asymmetrical. Usually, it does not produce head tremor. It has a frequency range 4 to 6 Hz.

### Cerebellar tremor

This is a postural, intention, or action tremor. It has a relatively low frequency in the range 3 to 4 Hz and is associated with ataxia and dysmetria.

### Writing tremor

This is not evident in other tasks requiring coordination, only during writing. It is considered a variant of focal hand dystonia (writer's cramp). It has no specific frequency range.

### Psychogenic tremor

This is an exclusion diagnosis. Symptoms vary in severity, depending on the subject's emotional state associated with stressful life events. Entrainment is a change in frequency of the tremor in adaptation to voluntary movements, such as a regular movement in the contralateral limb. It has not specific frequency range.

An accelerometer-based technique has been proposed as a proxy to measure and quantify tremors.

In addition to detecting tremors, it is known to attempt to compensate for the movement caused by tremors when a user holds a hand-held device, such as a spoon, cup or toothbrush. This typically involves driving a mass in an opposite direction to a tremor movement, thereby to create an impulse in a direction which causes damping of the tremor movement. However, this requires additional mass and associated drive components to be added to the device.

There is therefore a need for a simpler tremor compensation approach.

Anupam Pathak et. al., "A non-invasive handheld assistance device to accommodate essential tremor; A pilot Study" Movement Disorders vol. 29, no. 6, 27 May 2014 pp, 838 - 842 discloses a study of active cancellation of tremor. The tremor cancellation senses a direction of tremor and uses a motion-generating platform to provide cancellation.

WO 2020/205557 discloses an anti-tremor device, having a sensing unit for sensing tremor and a rotary control system which controls an inertia to eliminate or reduce the tremor.

WO 2016/133621 discloses a system for measuring tremor, having an inertial measurement unit within a handle of a hand-held tool.

### SUMMARY OF THE INVENTION

The invention is defined by the claims.

According to examples there is provided a hand-held personal care device comprising:
a handle;
an actuator for implementing a personal care function which generates a motion of a personal care element;
a drive signal generator for generating a drive signal for driving the actuator;
a sensor for generating a sensor signal which depends on motion of the personal care device; and
a processing arrangement, configured to:
   detect tremor vibrations from the sensor signal and thereby detect the presence of tremor and direction information in respect of the tremor; and
   adapt the actuator drive signal thereby to provide at least partial cancellation of the tremor.

The personal care function is the primary function of the personal care device.

This disclosure provides a personal care device which has a driven actuator. The driving of the actuator is able to generate an asymmetric motion such that a net force results, in a selected direction or set of directions. By controlling the actuator drive signal, the direction and optionally magnitude of this net force can be selected, and it is used to provide a resistance to the tremor vibrations, and thereby reduce the amount of tremor movement imparted to the device. It may be the motion of the personal care element which provides the tremor compensation, or it may instead or additionally be movement of an additional component, but driven by the same actuator, that provides the compensation.

The motion of the personal care element generated by the actuator for example comprises a rotation about a rotation axis in combination with a rocking of the rotation axis. The rocking, in particular, can be controlled to provide a force in a selectable direction. This type of motion is for example used in a toothbrush head, with rotation of the bristle field as well as rocking.

The processor is for example configured to adapt the actuator drive signal to provide asymmetry in the speed and/or distance of rocking of the rotation axis on opposite sides of a default axis.

In this way, different reaction forces need to be provided by the user in different directions. The larger reaction force acts to reduce the amount of tremor.

The actuator for example comprises a motor. The drive signal generator may then comprise an H-bridge circuit. The processor is then configured to control or drive the H-bridge circuit. For example, PWM pulses may be used, and they may be varied in pulse width, height and frequency. Different drive signals may be employed to generate imbalanced forces in a desired direction, using pulse width and amplitude variation or pulse shape variation or a combination of these. The resulting accelerations and decelerations give rise to different forces for which reaction forces must be provided by the user holding the device. The forces will thereby counter the tremor motion itself.

The device may further comprise a tremor compensation mass, wherein the actuator is for driving the personal care element and for driving the tremor compensation mass. This tremor compensation mass for example comprises a tapping plate. Thus, the drive train may be modified mechanically as well as electrically driven, in order to provide the desired tremor compensation function.

In one set of examples, the sensor comprises a sensor for sensing an actuator drive current. Monitoring the sensor current can be used to determine the tremor frequency, phase and direction.

In another set of examples, the sensor comprises a motion sensor, such as an inertial measurement unit, IMU. This can track motion and orientation of the device, and tremor vibrations can be detected within the motion sensing signals.

In another set of examples, the personal care function generates a vibration, and the sensor is for detecting vibration.

This approach is based on the recognition that when a user who suffers from tremor holds such a device, the lower frequency tremor vibrations are superposed with, or dampen, the vibrations generated by the actuator. The result is that tremor vibrations can be detected using a low-cost sensor, and in particular more simply than detecting the tremor motion pattern. In particular, the vibrating device amplifies the system response to the tremor on specific frequencies such as harmonics of the vibration function base frequency, due to an interaction between the function actuation and the tremor motion. These specific responses are due to the non-linearity of the system.

The sensor may then comprise a single axis vibration sensor. Another example is an actuator current sensor. In this latter case, the tremor vibrations are detectable as they provide a load to the actuator which is detectable in the actuator drive current. Thus, the tremor vibrations induce a system response which influences the actuator current. The characteristic frequencies of those vibrations can thereby be detected from the actuator current.

In one example, based on a single axis vibration sensor, the sensor comprises a microphone or accelerometer. This provides a simple low-cost sensing approach. In the case of a single axis sensor, the direction of the single axis sensor is known, so that vibrations are detected in that direction. By placing the sensor in the direction of the actuator, compensation can be provided in that direction. In a more complex harmonic form, a vibration originating from a different direction will be present in harmonic signals, and the harmonic can also be compensated to damp the original tremor motion.

In the case of detection of motor currents, when the motor is constructed using multiple coils, individual coil currents and loads can be measured, and the tremor direction can then be derived. The tremor can then be compensated by driving the more complex motor coils for the different directions. When the motor only has one coil to control, the direction will be limited to that motor and actuator direction both for detecting and compensating.

In addition to detecting and compensating for tremor, the processing arrangement may also be used to classify a detected tremor as one of a set of different types of tremor. This provides useful information to the user or medical professionals. The different tremor types for example have different frequency characteristics, but they may also have different temporal characteristics (i.e., amplitudes over time). For example, a tremor may be characterized as orthostatic, physiologic, essential tremor, Parkinsonism or cerebellar based at least in part on the dominant tremor frequency.

The processing arrangement may also be configured to determine a severity level of a detected tremor. This is based on a detected tremor amplitude and optionally duration information. The processing arrangement is for example configured to determine a progression of a detected tremor over time.

In one arrangement, the processing arrangement comprises a band pass filter arrangement for detecting the tremor vibrations. This provides a simple circuit solution.

The band pass filter arrangement for example comprises a first set of band pass filters within an expected frequency range of the tremors and a set of further band pass filters in a frequency range of higher harmonics of the expected frequency range of the tremors. Thus, the filters of the first set select different tremor types, but the detection is improved using detection of harmonics as well.

The band pass filter arrangement for example further comprises a further blocking filter for blocking an operating frequency of the vibration generated by the actuator.

In another arrangement, the processing arrangement comprises a neural network detect tremor vibrations. The neural network is for example supplied with a spectrogram of the sensor signal for a continuous range of frequencies up to at least double the personal care function vibration frequency. Thus, at least some harmonics are included in the spectrogram. This enables more accurate detection, and it may use the effect of the tremor vibration frequency and strength on the full frequency spectrum over a small period of time.

The device for example comprises a powered toothbrush or a shaver.

This disclosure also provides a method of compensating for tremors during use of a hand-held personal care device, which device implements a personal care function comprising driving motion of a personal care element using an actuator, wherein the method comprises:
generating a drive signal for driving the actuator;
sensing motion of the personal care device;
detecting tremor vibrations from the sensed motion and thereby detecting the presence of tremor and direction information in respect of the tremor; and
adapting the actuator drive signal thereby to provide at least partial cancellation of the tremor.

The disclosure also provides a computer program comprising computer program code means which is adapted, when said program is run on a computer, to implement the method defined above.

The disclosure also provides a computer program comprising computer program code means which is adapted, when said program is run on a computer, to implement the method defined above.

These and other aspects will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example of an electric toothbrush which detects and compensates for tremor in the user;
Fig. 2 shows H-bridge motor steering;
Fig. 3 shows a typical pulse width modulation signal for a bidirectional control signal;
Fig. 4 shows that a signal range has a DC offset caused by unequal positive and negative pulse widths;
Fig. 5 shows more clearly a positive offset caused by larger positive pulse widths than negative pulse widths;
Fig. 6 shows a positive offset based on pulse amplitude variation, in particular caused by larger amplitude positive pulses than negative pulses;
Fig. 7 shows an offset based on pulse shape variation;
Fig. 8 schematically shows imbalanced drive signals being applied to the four drive units of an H-bridge control circuit.
Fig. 9 shows how such an asymmetric signal may be applied to a toothbrush;
Fig. 10 shows a toothbrush side view and top view of the head motion; and
Fig. 11 shows a tapping plate and its control parts.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a hand-held personal care device having an actuator for implementing a personal care function, which generates a motion of a personal care element. Motion of the personal care device is sensed, and the presence and direction of tremor movements is detected. An actuator drive signal is adapted so as to provide at least partial cancellation of the tremor.

The invention may be applied to any personal care device which generates a motion when performing the personal care function. The personal care function may be a function for promoting health or for treating a medical condition or it may be part of a personal hygiene routine.

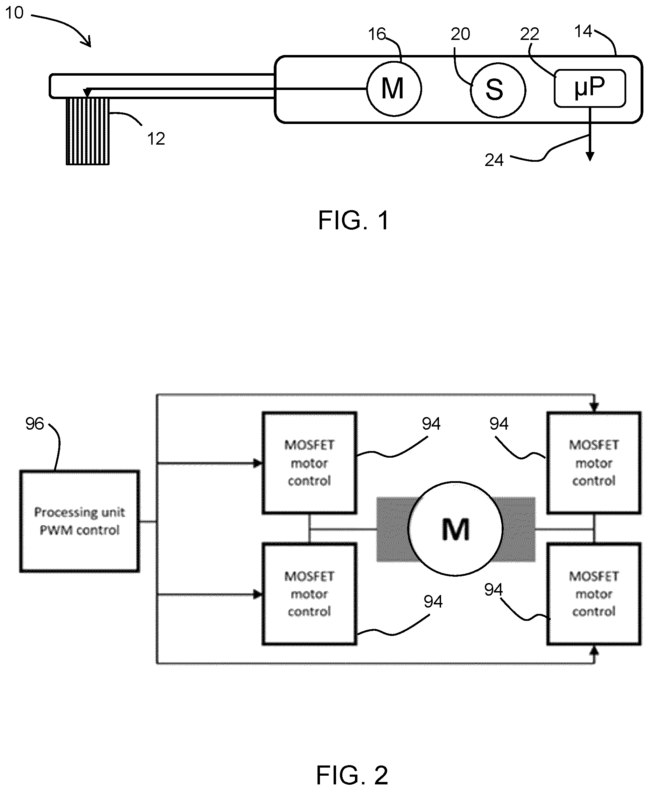

Fig. 1 shows an example of an electric toothbrush 10 having a driven brush head 12 (which is an example of a "personal care element for performing a personal care function") and a handle 14, with an actuator 16 in the handle for driving the head 12. The actuator in one example is a motor and the head is driven at least to rotate, and this generates a vibration which is transmitted to the device body. A sensor 20 generates a sensor signal which depends on the motion (e.g., vibration or vibration pattern) of the toothbrush.

A processing arrangement, schematically shown as processor 22, detects tremor vibrations from the sensor signal and thereby detects tremor. The detection of the tremor is used to control the drive signals applied to the actuator 16 and hence control the driven motion of the brush head 12. The driven motion of the head is configured to provide damping of the tremor vibrations. In particular, larger forces are applied to the user's hand (for which the user must deliver a reaction force) in a direction opposite to the direction of tremor motion that in a same direction as the tremor motion.

The motion of the personal care element is cyclic and has a higher cyclic frequency than the tremor vibration frequencies. As a result, the personal care element movements within multiple cycles can be modulated depending on the slower tremor vibrations.

The invention requires the detection of tremor, and then provides an adaptation to the actuator drive signal in order to provide compensation, i.e., partial cancellation, of the tremor motion.

The motion may be detected in known manner by using an inertial measurement unit, IMU. The IMU typically comprises a combination of a three-axis accelerometer and a three-axis gyroscope. This can measure the direction and magnitude of all motions.

The characteristic movement of the device in response to the actuator drive signals are typically in a much higher frequency band than the motions caused by tremor, so they can easily be distinguished. Thus, the tremor motions may be extracted from the overall motion of the device, even while the device is being used to perform its personal care function (in this example, the user is using the device to brush their teeth).

The manual movement of a toothbrush could however be close to the tremor motion. During testing, these motions may be differentiated.

In addition to providing tremor compensation, an output 24 may also be generated, as shown in Fig. 1, which may indicate when tremor has arisen, or more preferably it may identify different types of tremor and monitor progression of tremor symptoms over time. These options are discussed below.

Simpler sensing than the 6-axis IMU mentioned above may instead be used. Some options of sensing approaches will first be described, and then the cancellation function will be explained.

One option for the simpler sensing is that the sensor comprises a motor current sensor. In this case, the tremor vibrations, which are applied to the handle by the user, act as a load to the motor and the change in load modulates the motor current, by which is meant dampen or otherwise influence the existing vibrations of the personal care function and thereby alter the current. Thus, the motor current may be interpreted to determine the presence of, and more preferably the type of, tremor of the subject holding the toothbrush 10. In contrast to full 3 axis motion-based methods, another example is a single axis vibration sensor. The single axis vibration sensor may be implemented as an accelerometer or microphone.

The tremor is a complex motion and the vibrating device is also a complex system. As a result, the direct or indirect influence of any tremor motion direction can be detected. Indeed, the tremor motion itself will not be confined to a single direction. Of course, motion in the direction of a single axis sensor will be most sensitive.

Tremors can be identified from the sensor signal in general by detecting or filtering out characteristic frequency bands from the vibration frequencies of the toothbrush present in the sensor signal. An electric toothbrush for example operates with a rotation frequency around 70 to 80Hz, and a sonic toothbrush for example operates at around 260Hz, whereas the specific tremor frequencies lie in the range 3 to 20 Hz. Instead of filtering functions, power spectral analysis (using a FFT) and/or spectrograms may be employed.

The example above is based on monitoring motor currents. The motor current is sensitive in all directions it drives.

Another example of sensing approach uses a sensor in the form of a single axis accelerometer. The head of a powered toothbrush and its handle are usually integrated with many sensors such as an inertial measurement unit, IMU, camera, pressure, pain sensors etc. The IMU sensor provides an accelerometer signal during a brushing activity. The high frequency accelerometer signal can then be processed, optionally without low pass filtering, to derive the single axis sensing. The IMU is normally used for lower speeds to detect 3D motion and position tracking.

No matter how the tremor vibrations are sensed, the invention uses the sensed information relating to the presence and direction of tremors to implement a compensation i.e., cancellation function.

The invention in particular provides modification to the drive signal applied to the actuator of the device. The aim is to adapt the motion of the driven unit (e.g., toothbrush head) so that it drives the driven unit with an asymmetric motion. This asymmetric motion generates an impulse in a direction opposite to the direction of a tremor movement of the user of the device. This impulse is for example delivered at each drive cycle of the actuator.

There are two basic ways to reduce or even compensate tremors by such a drive train modification.

A first approach is a software approach. Providing a firmware adaptation to the way the actuator is driven, in particular by providing electronic modification of the drive train signal, can be used to generate asymmetric motion. Conceptually, the inverse of the tremor motion is applied to the motor.

The drive modification for example involves adding a temporary DC offset to a periodic bipolar drive signal to imbalance the motion. This approach is particularly effective if the motion generated by the actuator has an element of non-linearity. For example, a simple circular motion of a rotationally balanced driven element will enable only limited control using compensating impulses. For the example of a rotation, the compensation can be implemented within a range of directions over the angle of the rotation by driving the motor in an imbalanced way over this angle range.

However, if the driven element introduces directional vibrations in its normal operation, for example because there are multiple superposed movement trajectories, the direction and magnitude of these vibrations may be controlled by controlling the drive train signal. This makes the compensation range of directions larger.

By way of example, a toothbrush head may be driven with a rotation about a rotation axis in combination with a rocking of the rotation axis. The actuator drive signal may then be controlled to provide asymmetry in the speed and/or distance of rocking of the rotation axis on opposite sides of a default axis. In this way, a vibration component can be steered and have its magnitude controlled.

The rotation is typically back and forth (such as a rotation-oscillation motion or a a sonic low-amplitude angular sweeping motion). The sweeping motion may for example be combined with an (out of-phase) tapping motion (e.g., up/down).

The compensation must be within the stroke and speed limits of the system. The imbalanced inertia generated by the speed difference causes the required imbalanced forces. The DC offset may be used to select the motor position at which the speed difference change is implemented. Because the tremor is a changing repeating motion over time, compensation (i.e., damping) is by moving the system in the opposite direction with the same speed and inertia.

A second approach is a hardware approach, by which a mechanical modification of the drive train (drive shaft) is used to generate asymmetric motion, in combination with control of the actuator drive signal. This for example involves adding a tapping plate for generating an out-of-phase, out-of-plane motion.

The first approach will first be described, with reference to the example above of a power toothbrush. A power toothbrush motor is often controlled by a microcontroller via pulse width modulation and so-called H-bridge motor steering as shown in Fig. 2.

The H-bridge comprises a set of switches 94 controlled by a PWM controller 96. The controller controls the direction and speed of the motor by selectively turning the series of switches on and off.

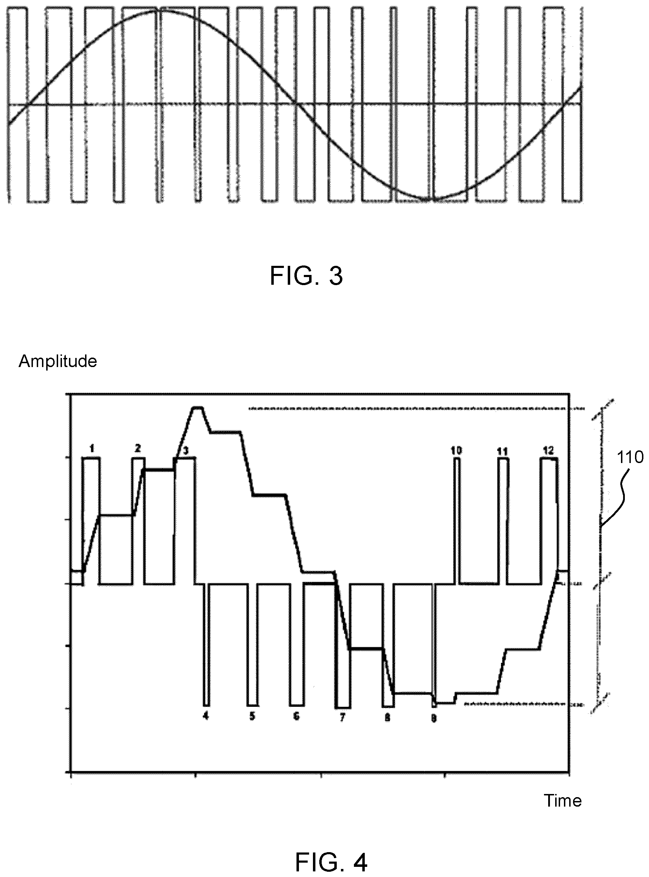

Fig. 3 shows a typical pulse width modulation signal for a bidirectional control signal. In this example, it can be seen that the control signal is balanced, evenly divided between the positive and negative values, resulting in a sinusoidal shape and hence motion. The width of each pulse is proportional to the signal level of the equivalent sinusoidal analog waveform. The analogue equivalent waveform is basically a low pass filtered version of the PWM signal, thus removing the frequencies of the PWM modulation. The PWM controller varies the pulse width of the signal to produce the desired output signal, which may be balanced or unbalanced.

Figs. 4 to 6 show pulse width modulation examples.

Fig. 4 shows that the signal range 110 has a DC offset caused by the unequal positive and negative pulse widths.

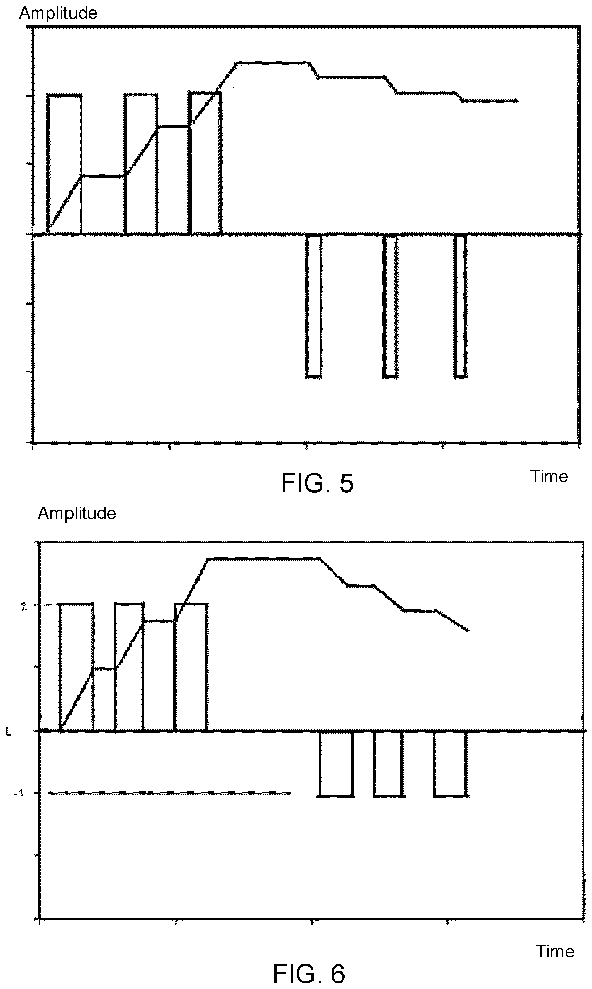

Fig. 5 shows more clearly a positive offset caused by larger positive pulse widths than negative pulse widths.

Fig. 6 shows a positive offset based on pulse amplitude variation, in particular caused by larger amplitude positive pulses than negative pulses.

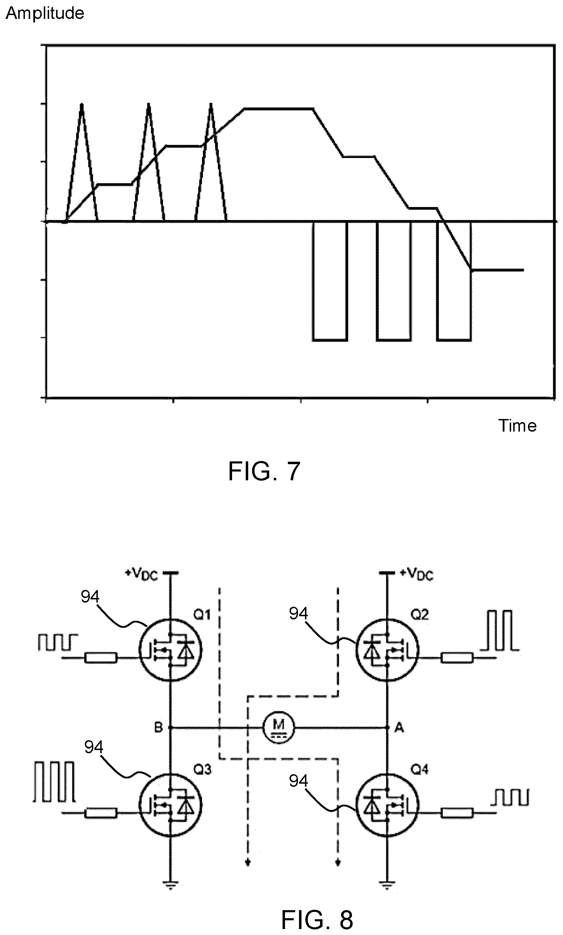

Another option is to adapt the pulse shape as shown in Fig. 7. The shape of the pulses can be changed, for example to a sawtooth shape as shown. In this way, the rate of change of actuator signal can also be controlled. A combination of different modulation techniques can be used.

As mentioned above, one drive circuit for controlling motor rotation is an H-bridge circuit.

Fig. 8 schematically shows imbalanced drive signals being applied to the four control units 94 of an H-bridge control circuit. One direction is controlled with much stronger signals (i.e., larger width and/or larger amplitude), causing the motor to move faster to one side.

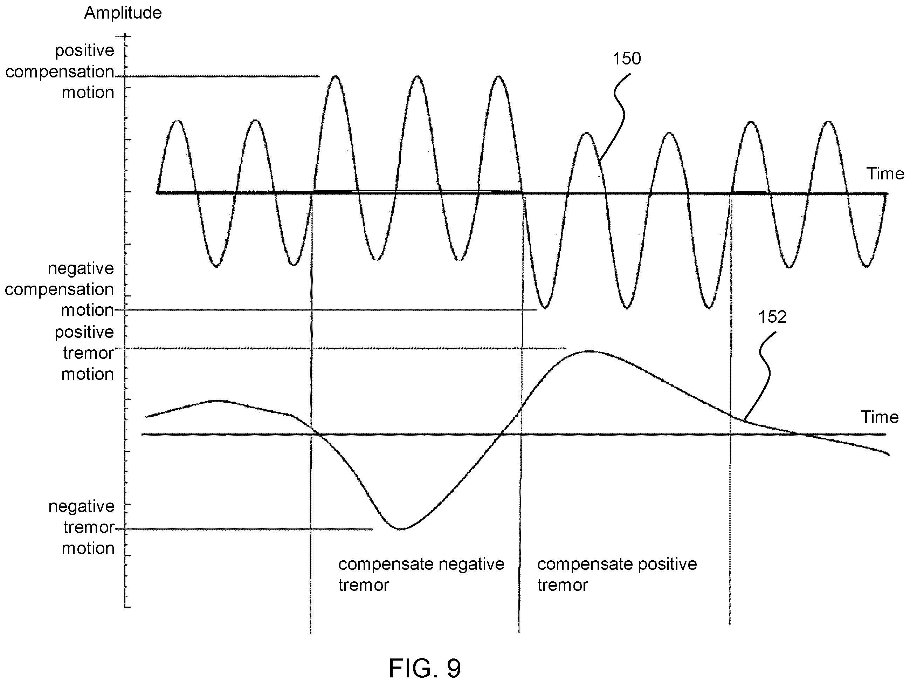

Fig. 9 shows how such an asymmetric signal may be applied to a toothbrush.

The actuation signal is shown as plot 150 and the detected tremor signal is shown as plot 152. The tremor motion for example has a frequency less than 20Hz whereas the brush motion is controlled at 260Hz (the vibrations are not shown to scale in Fig. 15 in that the frequency of the actuation signal is reduced to make the oscillations more clearly visible). The brush motion for example comprises 15 degrees rotation about a given axis.

As can be seen, the tremor is compensated and hence dampened by temporary and anti-phase unbalancing the motion of the toothbrush. The frequency of the brush motion stays constant in this example. As a result, the brush motion is modulated with an anti-phase tremor motion. It is noted that in Fig. 16 the terms "positive" and "negative" are used to indicate the direction of motion.

The higher frequency control of the toothbrush head enables the toothbrush head control signal to track the tremor motion.

In a typical example, the control of the H-bridge circuit may be used to control a direction and magnitude of compensating vibration in the plane of the toothbrush head. In particular, the tremor motion and rotation lie in the same plane (i.e., the axis of rotation is perpendicular to the general plane in which tremor motions exist). This is realistic, in that a hand tremor is typically side to side, hence in the plane of the toothbrush head when the toothbrush is held in a conventional manner.

This toothbrush head plane is generally defined as the x-y plane, with the x-axis parallel to the length direction of the brush head, and the y-direction across the length direction of the brush head. The z-direction is normal to the face of the toothbrush head.

The tremor vibrations, and hence the required compensatory vibrations are generally aligned with the y-axis.

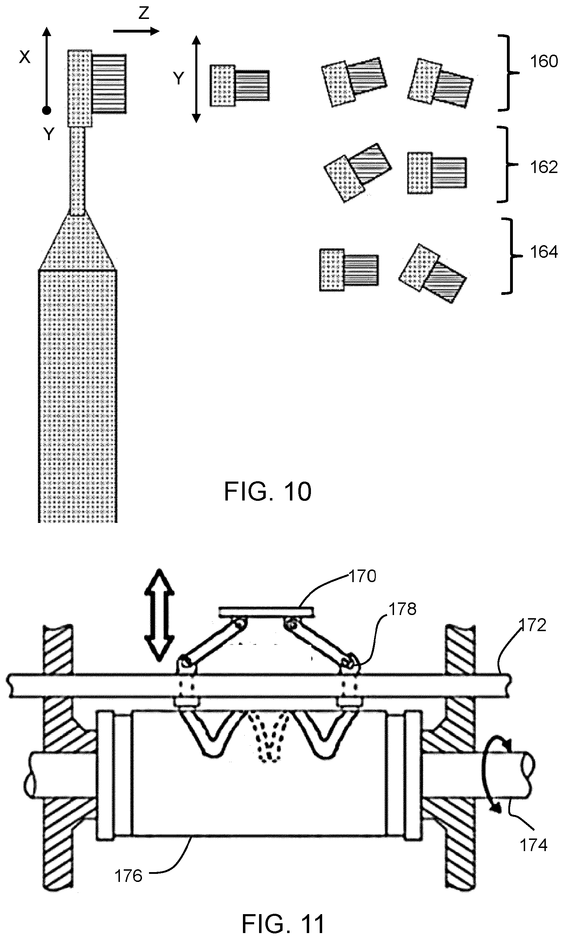

Fig. 10 shows a toothbrush side view and top view of the head motion.

One example of known motion provides a 7-degree rotation (i.e., rocking) of the head each side of the normal to the toothbrush head, in addition to a circular motion of the head. This rocking is about an axis parallel to the length axis of the toothbrush head, hence side-to-side across the y-axis. This is shown by the plots 160.

Unbalanced motion about the y-axis is shown by plots 162 and 164 (in opposite directions). The imbalance that is introduced compensates for tremor in the same x-y plane, in particular in the y-axis direction.

If a power toothbrush has electronically controlled motion in other directions than the y-axis, then tremor compensation may also be provided in other directions.

As explained above, a second approach is a hardware approach, by which a mechanical modification of the drive train (drive shaft) is used to generate asymmetric motion, in combination with control of the actuator drive signal. For instance, a DC offset can be used to drive the configuration based on the sensed tremor signal direction.

The mechanical modification of the drive train shaft for example involves adding a tapping plate for generating an out-of-phase, out-of-plane motion.

Fig. 11 shows a tapping plate 170 coupled to a fixed sliding shaft 172 by hinged joints. A shape slider 176 is attached to a sweeping shaft 174. Sliders 178 must follow a profile in the shape slider 176, so that as the sweeping shaft 174 rotates, the tapping plate 170 moves up and down.

The frequency and phase of the movement of the tapping plate will depend on the profile of the shape slider and the rotation of the sweeping shaft. Thus, control of the actuation of the sweeping shaft can be used to control the resulting forces and timings generated by the tapping plate. The tapping plate acts as counter mass to further reduce tremors, when asymmetrically driven as explained above.

By way of example, WO 2023/088721 discloses a system in which a second cleaning motion is generated with a tapping plate to optimize cleaning performance. The tapping plate is in this example connected to the bristle field, whereas the tapping plate in the system of the present invention is used as a passive counter mass, generating an inertia mass counter-momentum. Of course, the present system can also use the tapping element for both reducing tremors and providing a second cleaning motion.

By combining different motions and directions, a range of compensating motions can be created. When driving the motor with a specific pattern, the motion will peak in a specific direction and thus the compensating force will also peak in a specific direction. The taping mechanism provides an additional compensation direction, and thus enables a larger area for compensation.

In the example above, the processor is shown as part of the toothbrush. Of course, the signal processing may be performed remotely, and the personal care device may transmit data to the remote processing system to perform the analysis and return the results.

Various sensing and compensating options have been discussed above. In a most basic implementation, only the tremor motion or harmonics in the direction of a sensor and actuator are detected and compensated. When the motion is more complex, like the rotational motion of a power toothbrush, the direction of sensing and compensation will have a limited range over the angle of rotation.

The sensing can be achieved with a single axis approach because the tremor will be visible over a larger angle due to the complex non-linear vibrating system. Thus, the direction is detectable using a single axis sensor such as an accelerometer or microphone. When sensing actuator (motor) current, the motor current is directly coupled to the angular motion so will capture over the whole range the tremor motion. There is thus no need to reconstruct full 3D motion of the device. However, if full tremor 3D motion is sensed, this could enable compensation in all directions, but in practice there is typically a limited set of possible compensation directions so a full 3D reconstruction is not needed.

The invention has been described with reference to a toothbrush, but the same concept may be applied to any hand-held device which is actuated such that it vibrates in use, and the invention involves controlling the driven vibrations to compensate for detected tremor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner (optional)

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A hand-held personal care device comprising:
a handle (14);
a personal care element for performing a personal care function;
an actuator (16) for implementing the personal care function which generates a motion of the personal care element;
a drive signal generator for generating a drive signal for driving the actuator;
a sensor (20) for generating a sensor signal which depends on motion of the personal care device; and
a processing arrangement (22), configured to:
detect tremor vibrations from the sensor signal and thereby detect the presence of tremor and direction information in respect of the tremor; and
adapt the actuator drive signal thereby to provide at least partial cancellation of the tremor.

2. The device of claim 1, wherein the motion of the personal care element generated by the actuator comprises a rotation about a rotation axis in combination with a rocking of the rotation axis.

3. The device of claim 2, wherein the processor is configured to adapt the actuator drive signal to provide asymmetry in the speed and/or distance of rocking of the rotation axis on opposite sides of a default axis.

4. The device of any one of claims 1 to 3, wherein the actuator comprises a motor.

5. The device of claim 4, wherein the drive signal generator comprises an H-bridge circuit and the processor is configured to generate a set of control signals for control units of the H-bridge circuit.

6. The device of any one of claims 1 to 5, further comprising a tremor compensation mass, wherein the actuator is for driving the personal care element and for driving the tremor compensation mass.

7. The device of any one of claims 1 to 6, wherein the sensor (20) comprises a sensor for sensing an actuator drive current.

8. The device of any one of claims 1 to 6, wherein the sensor (20) comprises a motion sensor.

9. The device of claim 8, wherein the sensor (20) comprises a vibration sensor in the form of a microphone or single axis accelerometer.

10. The device of any one of claims 1 to 9, wherein the processing arrangement comprises a band pass filter arrangement for detecting the tremor vibrations.

11. The device of any one of claims 1 to 9, wherein the processing arrangement comprises a neural network detect tremor vibrations.

12. The device of claim 11, wherein the neural network is supplied with a spectrogram of the sensor signal for a continuous range of frequencies up to at least double the personal care function vibration frequency.

13. The device of any one of claims 1 to 12 comprising a powered toothbrush

14. A method of compensating for tremors during use of a hand-held personal care device according to any of claims 1 to 13, which device implements a personal care function comprising driving motion of a personal care element using an actuator, wherein the method comprises:
generating a drive signal for driving the actuator;
sensing motion of the personal care device;
detecting tremor vibrations from the sensed motion and thereby detecting the presence of tremor and direction information in respect of the tremor; and
adapting the actuator drive signal thereby to provide at least partial cancellation of the tremor.

15. A computer program comprising computer program code means which is adapted, when said program is run on a processor of a hand-held personal care device of claim 1, to implement the method of claim 14.

## Patentansprüche

1. Handgeführte Körperpflegevorrichtung, die Folgendes umfasst:
einen Griff (14);
ein Körperpflegeelement zur Durchführung einer Körperpflegefunktion;
einen Aktuator (16) zur Implementierung der Körperpflegefunktion, der eine Bewegung des Körperpflegeelements erzeugt;
einen Antriebssignalgenerator zum Erzeugen eines Antriebssignals zum Antreiben des Aktuators;
einen Sensor (20) zur Erzeugung eines Sensorsignals, das von der Bewegung der Körperpflegevorrichtung abhängt; und eine Verarbeitungsanordnung (22), die für Folgendes ausgelegt ist,
Erkennen von Tremorvibrationen aus dem Sensorsignal und dadurch Erkennen des Vorhandenseins eines Tremors sowie Informationen über die Richtung des Tremors; und
Anpassen des Antriebssignals des Aktuators dementsprechend, um so den Tremor zumindest teilweise aufzuheben.

2. Vorrichtung nach Anspruch 1, wobei die durch den Aktuator erzeugte Bewegung des Körperpflegeelements eine Drehung um eine Drehachse in Kombination mit einer Schaukelbewegung der Drehachse umfasst.

3. Vorrichtung nach Anspruch 2, wobei der Prozessor so konfiguriert ist, dass er das Antriebssignal für den Aktuator so anpasst, dass eine Asymmetrie hinsichtlich der Geschwindigkeit und/oder des Schaukelwegs der Drehachse auf gegenüberliegenden Seiten einer Standardachse entsteht.

4. Vorrichtung nach einem der Ansprüche 1 bis 3, wobei der Aktuator einen Motor umfasst.

5. Vorrichtung nach Anspruch 4, wobei der Antriebssignalgenerator eine H-Brücken-Schaltung umfasst und der Prozessor so konfiguriert ist, dass er einen Satz von Steuersignalen für die Steuereinheiten der H-Brücken-Schaltung erzeugt.

6. Vorrichtung nach einem der Ansprüche 1 bis 5, die ferner eine Tremorausgleichsmasse umfasst, wobei der Aktuator zum Antreiben des Körperpflegeelements und zum Antreiben der Tremorausgleichsmasse dient.

7. Vorrichtung nach einem der Ansprüche 1 bis 6, wobei der Sensor (20) einen Sensor zum Erfassen eines Antriebsstroms eines Aktuators umfasst.

8. Vorrichtung nach einem der Ansprüche 1 bis 6, wobei der Sensor (20) einen Bewegungssensor umfasst.

9. Vorrichtung nach Anspruch 8, wobei der Sensor (20) einen Vibrationssensor in Form eines Mikrofons oder eines einachsigen Beschleunigungsmessers umfasst.

10. Vorrichtung nach einem der Ansprüche 1 bis 9, wobei die Verarbeitungsanordnung eine Bandpassfilteranordnung zum Erfassen der Tremorvibrationen umfasst.

11. Vorrichtung nach einem der Ansprüche 1 bis 9, wobei die Verarbeitungsanordnung ein neuronales Netzwerk umfasst, das Tremorvibrationen erkennt.

12. Vorrichtung nach Anspruch 11, wobei dem neuronalen Netzwerk ein Spektrogramm des Sensorsignals für einen kontinuierlichen Frequenzbereich bis mindestens zum Doppelten der Vibrationsfrequenz der Körperpflegefunktion zugeführt wird.

13. Vorrichtung nach einem der Ansprüche 1 bis 12, die eine elektrische Zahnbürste umfasst;

14. Verfahren zum Ausgleich von Tremor während der Verwendung einer handgeführten Körperpflegevorrichtung nach einem der Ansprüche 1 bis 13, wobei die Vorrichtung eine Körperpflegefunktion ausführt, die das Antreiben der Bewegung eines Körperpflegeelements mittels eines Aktuators umfasst, wobei das Verfahren Folgendes umfasst:
Erzeugen eines Antriebssignals zum Antreiben des Aktuators;
Erfassen der Bewegung der Körperpflegevorrichtung;
Erfassen von Tremorvibrationen anhand der erfassten Bewegung und dadurch Ermitteln des Vorhandenseins eines Tremors sowie von Richtungsinformationen bezüglich des Tremors; und
Anpassen des Antriebssignals des Aktuators dementsprechend, um so den Tremor zumindest teilweise aufzuheben.

15. Computerprogramm, das Mittel in Form von Computerprogrammcode umfasst, die so ausgelegt sind, dass sie, wenn
das Programm auf einem Prozessor einer handgeführten Körperpflegevorrichtung nach Anspruch 1 ausgeführt wird, das Verfahren nach Anspruch 14 implementieren.

## Revendications

1. Dispositif de soins personnels à main, comprenant :
une poignée (14) ;
un élément de soins personnels pour réaliser une fonction de soins personnels ;
un actionneur (16) pour mettre en œuvre la fonction de soins personnels qui génère un mouvement de l'élément de soins personnels ;
un générateur de signal d'entraînement pour générer un signal d'entraînement pour entraîner l'actionneur ;
un capteur (20) pour générer un signal de capteur qui dépend de mouvement du dispositif de soins personnels ; et
un agencement de traitement (22), configuré pour :
détecter des vibrations de tremblement à partir du signal de capteur et ainsi détecter la présence de tremblement et des informations de direction informationen ce qui concerne le tremblement ; et
adapter le signal d'entraînement d'actionneur pour ainsi fournir une annulation au moins partielle du tremblement.

2. Dispositif de la revendication 1, dans lequel le mouvement de l'élément de soins personnels généré par l'actionneur comprend une rotation autour d'un axe de rotation en association avec un balancement de l'axe de rotation.

3. Dispositif de la revendication 2, dans lequel le processeur est configuré pour adapter le signal de commande d'actionneur pour fournir une asymétrie de la vitesse et/ou distance de balancement de l'axe de rotation sur des côtés opposés d'un axe par défaut.

4. Dispositif de l'une quelconque des revendications précédentes 1 à 3, dans lequel l'actionneur comprend un moteur.

5. Dispositif de la revendication 4, dans lequel le générateur de signal de commande comprend un circuit à pont H et le processeur est configuré pour générer un ensemble de signaux de commande pour des unités de commande du circuit à pont H.

6. Dispositif de l'une quelconque des revendications précédentes 1 à 5, comprenant en outre une masse de compensation de tremblement, dans lequel l'actionneur est destiné à entraîner l'élément de soins personnels et à entraîner la masse de compensation de tremblement.

7. Dispositif de l'une quelconque des revendications précédentes 1 à 6, dans lequel le capteur (20) comprend un capteur pour détecter un courant d'excitation d'actionneur.

8. Dispositif de l'une quelconque des revendications précédentes 1 à 6, dans lequel le capteur (20) comprend un capteur de mouvement.

9. Dispositif de la revendication 8, dans lequel le capteur (20) comprend un capteur de vibration sous la forme d'un microphone ou d'un accéléromètre uni-axial.

10. Dispositif de l'une quelconque des revendications précédentes 1 à 9, dans lequel l'agencement de traitement comprend un agencement filtre passe-bande pour détecter les vibrations de tremblement.

11. Dispositif de l'une quelconque des revendications précédentes 1 à 9, dans lequel l'agencement de traitement comprend un réseau neuronal détectant des vibrations de tremblement.

12. Dispositif de la revendication 11, dans lequel un spectrogramme du signal de capteur pour une plage continue de fréquences jusqu'au moins le double de la fréquence de vibration de fonction de soins personnels est procuré au réseau neuronal.

13. Dispositif de l'une quelconque des revendications précédentes 1 à 12, comprenant une brosse à dents électrique.

14. Procédé de compensation de tremblements durant l'utilisation d'un dispositif de soins personnels à main selon quelconques des revendications 1 à 13, lequel dispositif met en œuvre une fonction de soins personnels comprenant un mouvement d'entraînement d'un élément de soins personnels en utilisant un actionneur, dans lequel le procédé comprend :
la génération d'un signal de commande pour commander l'actionneur ;
la perception d'un mouvement du dispositif de soins personnels ;
la détection de vibrations de tremblement à partir du mouvement détecté et ainsi la détection de la présence de tremblement et d'informations de direction en ce qui concerne le tremblement ; et
l'adaptation du signal de commande d'actionneur pour ainsi fournir une annulation au moins partielle du tremblement.

15. Programme informatique comprenant des moyens codes de programme informatique qui sont adaptés, lorsque ledit programme est exécuté sur un processeur d'un dispositif de soins personnels à main de la revendication 1, pour mettre en œuvre le procédé de la revendication 14.
